(19) Europäisches Patentamt
European Patent Office
Office européen des brevets

(11) Publication number: **0 357 223 A2**

(12) # EUROPEAN PATENT APPLICATION

(21) Application number: 89307653.9

(22) Date of filing: 27.07.89

(51) Int. Cl.5: C07D 401/14 , C08K 5/34

(30) Priority: 29.08.88 CS 5817/88

(43) Date of publication of application: 07.03.90 Bulletin 90/10

(84) Designated Contracting States: BE CH DE ES FR GB IT LI NL

(71) Applicant: VYSKUMNY USTAV CHEMICKEJ TECHNOLOGIE VYSKUMNA A VYVOJOVA ORGANIZACIA
Dimitrovova 34
Bratislava(CS)

(72) Inventor: Vass, Frantisek
No 16 Sevcenkova
Bratislava(CS)
Inventor: Danko, Peter
No 5 Gercenova
Bratislava(CS)
Inventor: Povazancova, Marta
No 23 Tbiliska
Bratislava(CS)
Inventor: Karvas, Milan
No 15 Gajova
Bratislava(CS)
Inventor: Caucik, Pavol
No 26 Salviova
Bratislava(CS)
Inventor: Durmis, Julius
Ozvoldikova 5
Bratislava(CS)
Inventor: Baloch, Alojz
No 22 Pri bielom Krizi
Bratislava(CS)
Inventor: Goghova, Marcela
No 6 Zikova
Bratislava(CS)
Inventor: Benovic, Bohumil
No 20 Sturova
Svit(CS)
Inventor: Zak, Karel
No 9 Banska
Nitra(CS)
Inventor: Masek, Jan
No 28 Landauova
Bratislava(CS)

(74) Representative: Ablewhite, Alan James et al
MARKS & CLERK 57/60 Lincoln's Inn Fields
London WC2A 3LS(GB)

(54) **New polyaminotriazines and a process of their preparation.**

(57) The invention relates to new polyaminotriazines of the general formula /I/:

EP 0 357 223 A2

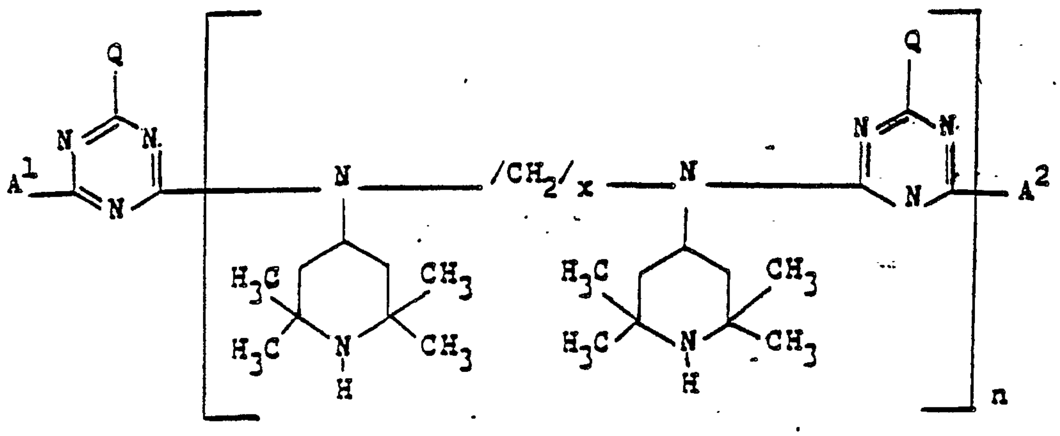

/I/

where Q, $A^1$ and $A^2$ are the same or different and mean the group $-NR^1R^2$, where $R^1$ is alkyl of 1 to 18 carbons, cyclohexyl or 2,2,6,6-tetramethyl-4-piperidyl; $R^2$ is hydrogen, alkyl of 1 to 12 carbons, cyclohexyl, and Q can also mean 1-piperidyl or 1-pyrrolidinyl; x is an integer from 2 to 12 and n is an integer from 2 to 20, which are efficient as polymer light stabilizers. The mentioned compounds are prepared by the reaction of the corresponding N,N-bis(2,2,6,6-tetramethyl-4-piperidyl)alkane diamine with 6-substituted 2,4-dichloro-1,3,5-triazine, which is used in the excess from 1 to 10 % mol at 140 to 220 °C in a hydrocarbon solvent, in the presence of the aqueous solution of an alkaline hydroxide under elevated pressure, or in the presence of a particulate alkaline hydroxide under the atmospheric pressure for 1 to 12 hours, to a chlorine-substituted oligomer which is treated under the same conditions with the corresponding amine.
The invention finds its use in the chemical industry.

## New polyaminotriazines and a process for their preparation

The invention relates to new polyaminotriazines and to the process of their preparation. The mentioned compounds are highly effective polymer light stabilizers.

The polyaminotriazines belong to one of the newest group of polymer light stabilizers. From the patent literature are known few types of these oligomers which are ended either by the one chlorine atom in the s-triazine ring or by a free amine group for their preparation used a bifunctional compound containing a tetraalkylpiperidine group / U.S. Patent 4 086 204, German Patent 2 636 144 /. U.S. Patent 4 331 586 covers the oligomers of which the s-triazine ring is always substituted by the one morpholine group.

The polyaminotriazines involving N,N′-bis/2,2,6,6-tetramethyl-4-piperidyl/alkane diamine units can be prepared either by the reaction of the cyanuric chloride with an excess of the corresponding N,N-bis/2,2,6,6-tetramethyl-4-piperidyl/alkane diamine, followed by the substitution of the last chlorine atoms / German Patents 3 113 455 and 3 117 964 / or by the direct oligomerization reaction of an excess of the corresponding N,N′-bis/2,2,6,6-tetramethyl-4-piperidyl/alkane diamine with a 6-substituted 2,4-dichloro-1,3,5-triazine / U.S. Patent 4 331 586, European Patents 45 721 and 93 693 / under or without pressure in the presence of a base. The known polyaminotriazines prepared by the processes mentioned above possess the significant disadvantage because of their relatively high content of the active chlorine which causes hygienic-toxicological problems.

The polyaminotriazines involving polyalkylpiperidine groups are highly effective polymer light stabilizers. They effectively inhibit nondesirable radical processes which take place during the photooxidation of hydrocarbon polymers. Disadvantages of low molecular weight compounds of this type are inherent first of all in their considerable volatility and extractibility, what is resulted in their subsequent loss from the utility polymer mainly with a big surface / thin foils, fibers / practically in the course of the processing or later during its storage / blooming /, dry cleaning or laundering, and by the exposure to atmospheric influences. However, the mentioned disadvantages can be significantly or totally eliminated by using the oligomeric aminotriazines involving polyalkylpiperidine groups with the optimum molecular weight / $\overline{M}_n$ = 2 000 to 5 000 / and suitable physical-chemical properties.

New polyaminotriazines of the general formula /I/ have been found:

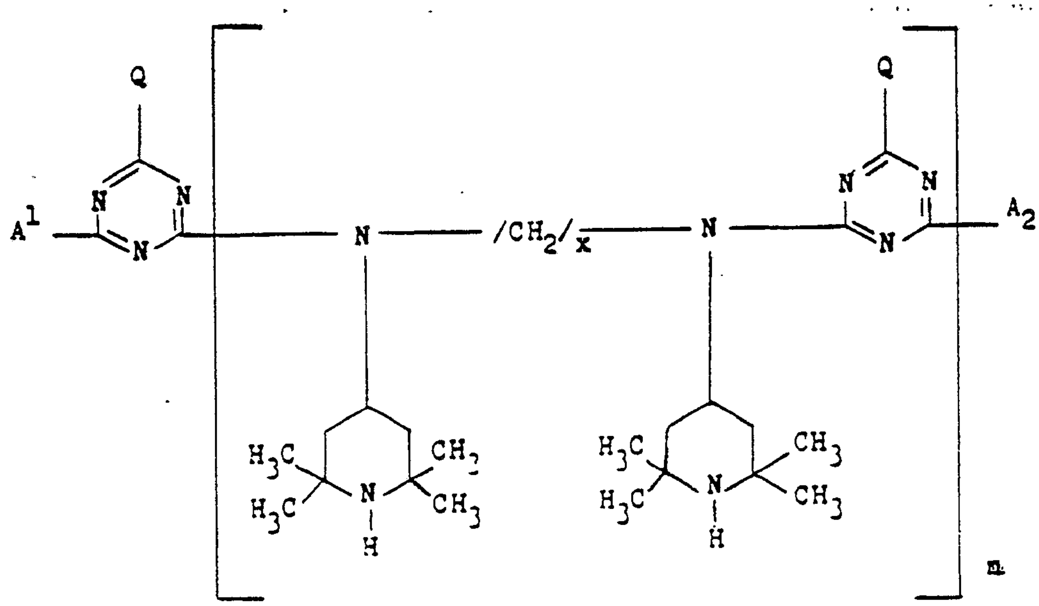

/I/

where Q, $A^1$ and $A^2$ are the same or different and they mean the group $-NR^1R^2$, where $R^1$ is alkyl of 1 to 18 carbons, cyclohexyl or 2,2,6,6-tetramethyl-4-piperidyl; $R^2$ is hydrogen, alkyl of 1 to 12 carbons, cyclohexyl, and Q can be meant also 1-piperidyl or 1-pyrrolidinyl; x is an integer from 2 to 12 and n is an integer from 2 to 20.

Subsequently, the process of the preparation of compounds of the general formula /I/ has been found by reacting of a N,N′-bis/2,2,6,6-tetramethyl-4-piperidyl/alkane diamine of the general formula /II/ :

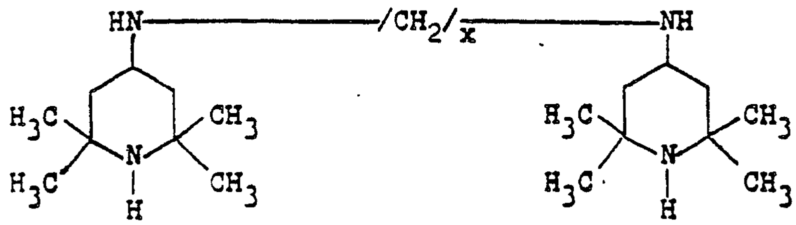

/II/

where x is of the meaning above, with a 6-substituted 2,4-dichloro-1,3,5-triazine of the general formula /III/ :

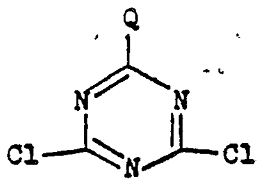

/III/

where Q is of the meaning above, which is used in the excess from 1 to 10 % by mol at 140 to 220 °C in a hydrocarbon solvent, in the presence of the aqueous solution of an alkaline metal hydroxide under elevated pressure or in the presence of a pulver alkaline hydroxide under the atmospheric pressure for 1 to 12 hours, followed in the formation of an oligomer of the general formula /I/ where Q, x and n are of the meaning above, and $A^1$ and $A^2$ mean chlorine, which is treated with an amine of the general formula /IV/ :

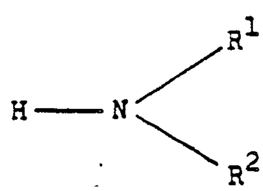

/IV/

where $R^1$ and $R^2$ are of the meaning above.

The polyaminotriazines of this invention of the general formula /I/ are useful as light stabilizers for thermoplastic substrates such as polyolefines, polyesters, polyethers, polyurethanes, polystyrenes, as films, fibers and the like. The therm "polyolefin" includes homopolymers of alpha olefins such as polyethylene, polypropylene, polybutadiene, polyisoprene, polystyrene, and the like, copolymers of alpha olefins such as ethylene-propylene copolymer, ethylene-butylene copolymer, ethylene-vinylacetate copolymer, styrene-butadiene copolymer, acrylonitrile-butadiene-styrene terpolymer, and the like. To the other organic materials susceptible to degradation by the effect of light, the properties of which are improved by the incorporation of the compounds of this invention of the general formula /I/, include natural and synthetic rubbers involving, for example, homo-, co-, and terpolymers of acrylonitrile, butadiene and styrene, and their blends.

The polyaminotriazines of this invention of the general formula /I/ are particularly useful in polyolefins, such as polyethylene, polypropylene, polybutylene, and like, and their copolymers.

The compounds of this invention of the general formula /I/ are incorporated to a polymer in the amount from 0,05 to 2,0% by weight, preferably from 0,1 to 1,0% by weight on the weight of an unstabilized polymer substrate.

The polymer compositions stabilized by polyaminotriazines of the general formula /I/ may contain also the other additives used, such as antioxidants, the other light stabilizers, flame retardants, antistatic agents, fillers, pigments, plasticizers, and the like.

Among antioxidants are particularly used the hindered phenols, such as, 2,6-di-tert. butyl-4-methylphenol and octadecyl-2-/3,5-di-tert. butyl-4-hydroxyphenyl/propionate, 1,3,5-tris/4-tert. butyl-3-hydroxy-2,6-dimethylbenzyl-s-triazine-2,4,6-/1H,3H,5H/-trione; esters of thiodipropionic acid, such as, dilauryl thiodipropionate and di-stearyl thiodipropionate; phosphites, such as triphenyl phosphite, trinonyl phosphite, diisodecyl pentaerythrityl diphosphite and diphenyl-decyl phosphite; and combinations thereof.

Among light stabilizers are particularly used the benzotriazoles; such as 2-/2-hydroxy-3,5-di-tert. oktylphenyl/benztriazole and 2-/2-hydroxy-3,5-di-tert.butylphenyl/-5-chlorbenztriazole; hydroxybenzo phenones, such as 2-hydroxy-4-methoxybenzophenone and 2-hydroxy-4-octyloxybenzophenone; hindered phenol esters, such as 2,4-di-tert. butylphenyl-3,5-di-tert.butyl-4-hydroxybenzoate; metal complexes, such as nickel salt of a monalkyl ester of the 4-hydroxy-3,5-di-tert.butylbenzylphosphonic acid.

The polyaminotriazines of this invention of the general formula /I/ may be incorporated into the polymer substrate by any of the known and for this purpose used techniques, such as by dry blending in the powder or granular form, followed by processing /milling/ in a Banbury mixer, by molding, injection molding or extruding. The oligomers of this invention of the general formula /I/ may be added to the polymer substrate/in the powder or granular form/ even as their solution in a suitable solvent, alternatively during the polymerization or before processing of the polymer to the products.

The polyaminotriazines of this invention of the general formula /I/ possess the excelent photostabilization activity, higher than known stabilizers of this type, very good compatibility with polymers, extremly low volatility and extractibility from polymers during laundering or dry cleaning, and the excelent long-term thermal stability. With respect to not involving the active chlorine, they possess very favourable hygienic-toxicological characters - they are practically nontoxic, not irritating the skin, and the eye-irritability is only short and in a minimum extent. By the process of this invention they can be prepared in the high purity and yield /over 99%/.

The following examples are illustrative but not limiting the claims of this invention.

Example 1

To a 100 ml pressure vessel were charged 7,9g /0,020 mol/ of N,N′-bis/2,2,6,6-tetramethyl-4-piperidyl/ hexane diamine, 5,95g / 0,0215 mol/ of 6-tert.octylamino-2,4-dichloro-1,3,5-triazine, 3,85g of 40% by weight of the aqueous solution of sodium hydroxide and 20 ml of xylene. The pressure vessel was closed and its content was heated at 180 °C for 8 hours under stirring while the pressure had arisen about to 0,7 MPa. After cooling the pressure vessel, to the reaction mixture were added 0,6g /0,004 mol/ of tert.octylamine, 0,45g of 40 % by weight of the aqueous solution of sodium hydroxide and 5 ml of xylene, and its content was again heated at 180 °C for 2 hours under vigorous stirring while the pressure had arisen again to the mentioned value. After completion of the reaction, the cooled reaction mixture was diluted with 50 ml of xylene and water was azeotropically distilled off. By the suction filtration was obtained the clear solution of the oligomer from which the solvent was driven off by the vacuum distillation. 11,9g /99,3 %/ of the white glassy, easily pulverable product was obtained after cooling. This represents the polyaminotriazine of the general formula /I/, where Q, $A^1$ and $A^2$ mean tert.octylamino group and x = 6, with the melting point of 119 to 134 °C and molecularweight of 3 800 /determined by the vapour pressure osometry/.

| Elemental Analysis for $C_{35}H_{66}N_8$ | |
|---|---|
| Calculated : | 18,78 % N |
| Found : | 18,87 % N |
| Chlorine content: | 0,00 % |

I.R. Spectrum /$CCl_4$/:
3 440 - $\gamma$/NH, free/ ; 2 950, 2860 - $\gamma_{as}$/C-H/, $\gamma_s$/C-H/; 1 560 -$\delta$/N-H/ ; 1 480 - $\delta$/C-H/; 1 370 + 1 360 dublet - $\delta_{as}$/gem. $CH_3$/ ; 1 310 - $\gamma$/C-N/ $cm^{-1}$. $^1H$ - NMR Spectrum /$CDCl_3$/:
0,96 - singlet /9 H, tert.butyl/ ; 1,1, 1,17 - dublet /24 H, gem. $CH_3$/ ; 1,27 - singlet /8 H, -$CH_2$- from the aliphatic chain/ ; 1,46 - singlet /14 H, -$CH_2$- ring, $CH_3$ - C - $CH_3$/ ;
0,5 - 2,1 - multiplet /2 H, N - H hindered/ ; 3,25 - broad band /4 H, - $CH_2$ - 1,3,5 triazine/ ;
4,75 - singlet /1 H - NH - 1,3,5-triazine/ ; 5,17 - broad band /2 H, CH - N from the ring/ ppm.

Example 2

To a 200 ml pressure vessel were charged 11,8 g/0,03 mol/ of N,N′-bis/2,2,6,6-tetramethyl-4-piperidyl/hexane diamine and 6,2 g of 50 % by weight of the aqueous solution of potassium hydroxide. The

pressure vessel was closed and by means of a pressure pump was added 10,6g /0,03225 mol/ of 6-dicyclohexylamino-2,4-dichloro-1,3,5-triazine in 20 ml of toluene at 190°C under vigorous stirring within 0,5 hour. The reaction mixture was heated at the mentioned temperature under continuing stirring for 6 hours while the pressure in the vessel had reached the value from 1,0 to 1,2 MPa. After that the mixture of 0,65g of 50 % by weight of the aqueous solution of potassium hydroxide, 0,57g /0,0045 mol/ of cyclohexylamine and 5 ml of toluene, was charged to the pressure vessel by using the pressure pump. The reaction was completed without changing the conditions within 2 hours,and after cooling the reaction mixture was worked out as in Example 1. The polyaminotriazine of the general formula /I/ was obtained, where Q was dicyclohexylamino group, $A^1$ and $A^2$ were cyclohexylamino groups and x was 6, in the form of a white glassy, easily pulverable solid with melting point between 170 to 198 °C and molecular weight of 3 900 /determined by the vapour pressure osmometry/. The yield was 19,5g /99,7 %/.

| Elemental Analysis for $C_{39}H_{70}N_8$ | |
|---|---|
| Calculated : | 17,21 % N |
| Found : | 17,44 % N |
| Chlorine content: | 0,00 % N |

I.R. Spectrum /$CCl_4$/ :
3 400 - $\gamma$/NH, free/ ; 2 930 - $\gamma_{as}$/C-H/, $\gamma_s$/C-H/ - 2 860, 1 530 -- $\delta$ /N-H/; 1 470 - $\delta$/C-H/; 1 370 + 1360 dublet - $\delta_s$/gem. $CH_3$/ ; 1 310 -$\gamma$/C-N/ $cm^{-1}$.
$^1$H - N.M.R. Spectrum /$CDCl_3$/ :
1,11, 1,16 -dublet /24 H, gem. $CH_3$/; 1,26 - singlet /8 H, - $CH_2$ -from the aliphatic chain/;
0,5 - 2,3 - multiplet /20 H, N-H hindered, -$CH_2$- from the piperidine ring, - $CH_2$ - from the cyclohexane ring/;
3,25 - broad band /4 H, -$CH_2$-N-1,3,5-triazine/; 3,75 -broad band /1 H, - N - HC< from the cyclohexane ring/;
4,5 - singlet /1 H, -NH-1,3,5-triazine/; 5,2 - broad band /2 H, >CH-N from the piperidine ring/ ppm.

Example 3

To a 250 ml three neck round-bottom glass flask were charged 15,7g /0,04 mol/ of N,N-bis/2,2,6,6-tetramethyl-4-piperidyl/hexane diamine, 10,4g /0,042 mol/ of 6-cyclohexylamino-2,4-dichloro-1,3,5-- triazine, 3,08g of pulver sodium hydroxide, and 60 ml of tetraline. The flask was fitted with a condenser and stirrer with a KPG seal, and the reaction mixture was kept under a reflux and continuing stirring for 12 hours. The oligomer formed was after that left yet for further recting with 0,4g /0,0045 mol/ of cyclohexylamine, and the additional sodium hydroxide in the amount of 0,18g /0,0045 mol/ and 10 ml of tetraline under the same conditions for 4 hours. The cooled reaction mixture was suction filtrated and tetraline was driven off under the vacuum. 22,55g /99,12 % yield/ of a brownish glassy, easily pulverable product was obtained,which represented the polyaminotriazine if the general formula /I/, where Q, $A^1$ and $A^2$ meant cyclohexylamino groups and x = 6,with melting point between 168 to 186 °C and molecular weight of 4 800 /determined by the vapour pressure osmometry/.

| Elemental Analysis for $C_{33}H_{60}N_8$ | |
|---|---|
| Calculated : | 19,69 % N |
| Found : | 19,77 % N |
| Chlorine content: | 0,00 % N |

I.R. Spectrum /$CCl_4$/:
3 440 - $\gamma$/N-H, free/; 2 930, 2 860 - $\gamma_{as}$/C-H/, $\gamma_s$/C-H/; 1 520 - $\delta$/N-H/; 1 480 - $\delta$ /C-H/; 1 380 + 1370 dublet - $\delta_s$/gem. $CH_3$/; 1 310 - $\gamma$/C-N/ $cm^{-1}$.
$^1$H - N.M.R. Spectrum /$CDCl_3$/ :

1,12, 1,17 - dublet /24 H, gem. $CH_3$/; 1,26 - singlet /8 H, - $CH_2$ -from the aliphatic chain/; 0,5 - 2,8 - multiplet /20 H, -NH hindered, -$CH_2$- from the piperidine ring, -$CH_2$- from the cyclohexane ring/; 3,28- broad band /4 H, -$CH_2$-N- 1,3,5-triazine/; 3,75 - broad band /1 H, -N- CH from the cyclohexane ring/; 5,2 broad band /2 H, >CH -N from the piperidine ring/ ppm.

The other polyaminotriazines of this invention of the general formula /I/ were prepared by the methods according to the introduced examples, and characterized by the data in Table 1.

Table 1

| Example | Q | "X" | $A^1$ | $A^2$ | Method accord. to Example | Excess of sub. 2,4-dichloro-1,3,5-triazine /mol %/ | Melting point /°C/ | $M_n$ | Yield /%/ | Elemental analysis Calc. % N | Found % N | Found % Cl. |
|---|---|---|---|---|---|---|---|---|---|---|---|---|
| 1 | 2 | 3 | 4 | 5 | 6 | 7 | 8 | 9 | 10 | 11 | 12 | 13 |
| 4 | tert. octylamino | 6 | n-octyl-2,2-6,6-tetramethyl-4-piperidylamino | n-octyl-2,2-6,6-tetramethyl-4-piperidylamino | 1 | 7,5 | 125-140 | 3 350 | 99,4 | 18,78 | 18,89 | 0,0 |
| 5 | tert. octylamino | 6 | tert. octylamino | n-octadecylamino | 2 | 5 | 118-134 | 4 570 | 99,5 | 18,78 | 18,92 | 0,0 |
| 6 | tert. octylamino | 2 | tert. octylamino | tert. octylamino | 1 | 7,5 | 231-251 | 3 420 | 98,7 | 20,64 | 20,94 | 0,0 |
| 7 | tert. octylamino | 4 | tert. octylamino | tert. octylamino | 1 | 7,5 | 187-200 | 3 470 | 98,9 | 19,62 | 19,91 | 0,0 |
| 8 | tert. octylamino | 8 | tert. octylamino | cyclohexylamino | 1 | 7,5 | 204-220 | 3 700 | 99,4 | 17,87 | 18,02 | 0,0 |
| 9 | tert. octylamino | 10 | tert. octylamino | tert. octylamino | 1 | 7,5 | 153-162 | 3 900 | 98,6 | 17,11 | 17,45 | 0,0 |
| 10 | tert. octylamino | 12 | tert. octylamino | n-butylamino | 1 | 7,5 | 65-72 | 4 160 | 97,5 | 16,40 | 16,59 | 0,0 |
| 11 | cyclohexylamino | 6 | n-butyl-2,2-6,6-tetramethyl-4-piperidylamino | n-butyl-2,2-6,6-tetramethyl-4-piperidylamino | 2 | 5 | 180-196 | 4 800 | 99,5 | 19,69 | 19,91 | 0,0 |

Table 1 continued

| 1 | 2 | 3 | 4 | 5 | 6 | 7 | 8 | 9 | 10 | 11 | 12 | 13 |
|---|---|---|---|---|---|---|---|---|---|---|---|---|
| 12 | cyclohexyl-amino | 6 | 2,2,6,6-tetramethyl-4--piperidyl-amino | 2,2,6,6-tetramethyl-4-piperidylamino | 3 | 7,5 | 170-188 | 3 500 | 98,4 | 19,69 | 19,75 | 0,0 |
| 13 | cyclohexyl-amino | 6 | cyclohexyl-amino | cyclohexyl-amino | 1 | 5 | 172-186 | 4 750 | 99,3 | 19,69 | 19,83 | 0,0 |
| 14 | cyclohexyl-amino | 6 | cyclohexyl-amino | 1,6-bis/2,2,-6,6-tetramethyl-4-piperidylamino/hexane-N-yl | 1 | 2,5 | 170-176 | 6 200 | 98,9 | 19,69 | 19,91 | 0,0 |
| 15 | ethylcyclo-hexylamino | 4 | cyclohexyl-amino | n-butyl-2,2,-6,6-tetramethyl-4-piperidylamino | 3 | 5 | 159-171 | 4 960 | 98,3 | 19,69 | 19,86 | 0,0 |
| 16 | isopropyl-cyclohexyl-amino | 8 | cyclohexyl-amino | diethylamino | 1 | 7,5 | 140-159 | 4 100 | 99,1 | 17,81 | 18,09 | 0,0 |
| 17 | isopropyl-amino | 6 | isopropyl-amino | isopropyl-amino | 1 | 7,5 | 137-151 | 3 400 | 99,2 | 21,19 | 21,65 | 0,0 |
| 18 | diethylamino | 6 | diethylamino | diethylamino | 1 | 5 | 161-184 | 4 700 | 99,4 | 20,64 | 20,89 | 0,0 |
| 19 | n-octylamino | 6 | n-octylamino | n-octylamino | 1 | 7,5 | 114-125 | 3 700 | 99,1 | 18,78 | 18,97 | 0,0 |
| 20 | n-octadecyl-amino | 6 | n-octadecyl-amino | n-octadecyl-amino | 3 | 10 | 70-89 | 3 300 | 98,9 | 15,16 | 15,41 | 0,0 |
| 21 | diisopropyl-amino | 6 | cyclohexyl-amino | cyclohexyl-amino | 1 | 7,5 | 122-140 | 3 600 | 98,4 | 19,62 | 19,85 | 0,0 |

Table 1 continued

| 1 | 2 | 3 | 4 | 5 | 6 | 7 | 8 | 9 | 10 | 11 | 12 | 13 |
|---|---|---|---|---|---|---|---|---|---|---|---|---|
| 22 | tert. butylamino | 6 | tert. butylamino | tert. butylamino | 3 | 5,0 | 180-191 | 4 400 | 98,4 | 20,64 | 20,92 | 0,0 |
| 23 | 1-pyrrolidyl | 6 | cyclohexyl-amino | cyclohexyl-amino | 1 | 7,5 | 169-182 | 3 300 | 98,1 | 20,81 | 21,30 | 0,0 |
| 24 | 1-piperidyl | 6 | cyclohexyl-amino | cyclohexyl-amino | 1 | 7,5 | 178-194 | 3 200 | 98,6 | 20,28 | 20,73 | 0,0 |

Example 25

The compositions containing 0,1 % by weight of 2,6-di-tert.butyl-4-methylphenol, 0,15 % by weight of calcium stearate and 0,2 % by weight of a stabilizer tested were prepared from the unstabilized polypropylene of HPF type with the melt flow index $MFI_{230}$ = 11g/10 min. The individual compositions were homogenized in the chamber of the Brabender Plastograph in the nitrogen atmosphere at 190 °C within 10 minutes and 0,5 mm films were molded of them at 260 °C. In the same manner were prepared the films from the individual unstabilized polypropylene and polypropylene without a light stabilizer. For the comparison was used in this and the other tests /Example 24 to 28/, poly {[6-tert.octylamino-1,3,5-triazine-2,4-diyl]-[2-/2,2,6,6-tetramethyl-4-piperidyl/imino]- [4-/2,2,6,6-tetramethyl-4-piperidyl/imino]-hexamethylene} according to Eur. Pat. 93 693 as the commercial light stabilizer with the chlorine content of 0,4 % by weight. The samples were evaluated for the thermooxidative stability in a draft oven at 110 °C.
The results obtained are included in Table 2.

Table 2

| Stabilizer tested | Thermooxidative stability /hours/ |
|---|---|
| Individual unstabilized polypropylene | 40 |
| Polypropylene without a light stabilizer | 120 |
| Compound according to Example : 1 | 1 600 |
| 4 | 1 800 |
| 6 | 1 900 |
| 7 | 2 050 |
| 8 | 2 140 |
| 9 | 1 970 |
| 10 | 1 700 |
| 12 | 2 200 |
| 13 | 2 750 |
| Light Stabilizer according to E.P. 93 693 | 1 600 |

Example 26

In the case of the polypropylene compositions prepared according to Example 25, the photooxidation stability of the molded films of the thickness of 0,1 mm, prepared by repeated molding of 0,5 mm films at 200 °C, was investigated, too. The films were exposed in the Xenotest 450 at 90 °C for 50 hours before and after laundering in distilled water containing a detergent. The increase of the absorbance of carbonyl groups /$\Delta A_{CO}$/ in the polypropylene films was investigated by using IR spe trometer. The criterion of the effectiveness: the time to the increase of $\Delta A_{CO}$ = 0,3. The values measured are included in Table 3.

Table 3

| Stabilizer tested | Photooxidation stability - Xenotest 450,$\Delta A_{CO}$ = 0,3/hr/ | |
|---|---|---|
| | before and | after laundering |
| Individual unstabilized polypropylene | 200 | - |
| Polypropylene without a light stabilizer | 900 | 500 |
| Compound according to Example : 1 | 3 500 | 2 800 |
| 4 | 3 800 | 3 000 |
| 5 | 3 600 | 3 100 |
| 7 | 3 200 | 2 600 |
| 8 | 3 300 | 2 600 |
| 9 | 3 200 | 2 700 |
| 11 | 3 700 | 3 100 |
| 12 | 3 900 | 3 400 |
| 13 | 3 800 | 3 200 |
| Light stabilizer according to E.P. 93 693 | 3 200 | 2 500 |

Example 27

From the unstabilized polypropylene of HPF type with the melt flow index $MFI_{230}$ = 9,2 g/ 10 min. were prepared compositions with stabilizers which were granulated at 230 °C on the double extruder with the diameter of 35 mm. From the such prepared granulation product were extruded at 250 °C the films of the thickness of 50 µm by using the equipment "Plasticizer". The films comprised 0,1 % by weight of 2,6-di-tert.butyl-4-methylphenol, 0,05 to 2 % by weight of a light stabilizer and 0,15 % by weight of calcium stearate. The photooxidation stability of these films was investigated by the accelerated aging in the Xenotest 450 and measuring of the increase of carbonyl groups /$\Delta A_{CO}$/, using IR spectrometry. The evaluation was finished at the increase of the absorbance to the value of 0,3. Table 4 contains the results measured.

Table 4

| Stabilizer tested | Photooxidation stability - Xenotest 450 | |
|---|---|---|
| | % by weight | $\Delta A_{CO}$ = 0,3/hr/ |
| Polypropylene without a light stabilizer | - | 600 |
| Compounds according to Example : 1 | 0,20 | 4 000 |
| 13 | 0,05 | 1 050 |
| 13 | 0,10 | 2 100 |
| 13 | 0,20 | 3 300 |
| 13 | 0,50 | 6 700 |
| 13 | 1,00 | 10 400 |
| 13 | 2,00 | >12 000 |
| Light stabilizer according to E.P. 93 693 | 0,20 | 2 900 |

Example 28

From the unstabilized pulver polypropylene of HPF type with the melt flow index $MFI_{230}$ = 9,2 g/ 10 min. were in the same manner as in Example 25 prepared granules from which were produced fibers at 270 to 320 °C on the laboratory extruder with diameter of 32 mm. The fibers comprised 0,1 % by weight of 2,6-di-tert.butyl-4-methylphenol, 0,2 % by weight of a light stabilizer and 0,15 by weight of calcium stearate. The fibers were irradiated in the Xenotest 450 and the decrease of the strength was investigated. As the criterion of the evaluation was used the time of the irradiation /hours/ needed to the decrease of the original strength of fibers of 50 %. The results obtained are included in Table 5.

Table 5

| Stabilizer tested | Photooxidation stability /hr/ Xenotest 450 |
|---|---|
| Compound according to Example 1 | 1 200 |
| Compound according to Example 13 | 1 050 |
| Light stabilizer according to E.P. 93 693 | 900 |

Example 29

The unstabilized polypropylene / in the pulver form / of MPD type with the melt flow index $MFI_{230}$ = 2,4 g/ 10 min. was blended with 0,1 % by weight of 2,6-di-tert.butyl-4-methylphenol, 0,2 % by weight of a light stabilizer, 0,1 % by weight of calcium stearate and 0,05 % by weight of 2,5-dimethyl-2,5-di-tert.butylperoxy hexane / degradation agent /, and the compositions were processed into the granulation product as in Example 27. One portion of the granulation product was processed at 220 °C on the laboratory extruder into the films of the thickness of 50 µm and from the another portion were prepared the fibers at 240 to 250 °C on the laboratory extruder with the diameter of 32 mm. The films and fibers were exposed in the Xenotest 450 and their lifetime / hours / was determined. The results are in Table 6.

Table 6

| Stabilizer tested | Extruded films $\Delta A_{CO}$ = 0,2 /hr/ | Fibers 50 % of the original strength /hr/ |
|---|---|---|
| Compound according to Example 3 | 4 100 | 1 200 |
| Compound according to Example 13 | 4 200 | 1 250 |
| Compound according to Example 14 | 4 000 | 1 150 |
| Light stabilizer according to E.P. 93 693 | 4 000 | 1 000 |

Example 30

The unstabilized low-density polyethylene with the melt flow index $MFI_{190}$ = 2 g/10 min. was blended in the chamber of the Brabender Plastograph with 0,05 to 0,5 % by weight of a stabilizer tested and 0,1 % by weight of calcium stearate within 10 minutes at 160 °C in the nitrogen atmosphere. From the such prepared compositions were molded the films of the thickness of 0,5 mm. The unstabilized polyethylene was processed in the same manner. The films were exposed to the irradiation in the Xenotest 450. As the criterion of their lifetime was used the time /hours/ needed to the attainment of the increase of the absorbance of carbonyl groups to the value of 0,5. The values found are summarized in Table 7.

Table 7

| Stabilizer tested | % by weight | Xenotest 450 $A_{CO}$ = 0,5/hr/ |
|---|---|---|
| Unstabilized polyethylene | - | 1 700 |
| Compound according to Example: 1 | 0,20 | 4 600 |
| 3 | 0,20 | 3 500 |
| 11 | 0,20 | 3 300 |
| 12 | 0,20 | 3 450 |
| 13 | 0,05 | 1 800 |
| 13 | 0,10 | 2 300 |
| 13 | 0,20 | 4 000 |
| 13 | 0,50 | 6 000 |
| Light stabilizer prepared according to E.P. 93 693 | 0,20 | 3 350 |

Example 31

The unstabilized low-density polyethylene with the melt flow index $MFI_{190}$ = 2 g/10 min. was blended on the planetary mixer with 0,2 % by weight of a stabilizer tested and 0,1 % by weight of calcium stearate. The such prepared compositions were processed on the screw of the Brabender Plastograph at 160/170/180/175 °C and 5 rpm into the granulate from which were formed with blown films by using the same equipment at 200/210/220/215 °C and 40 rpm. As the criterion of the lifetime of the films was used the time /hours/ needed for the attainment of the increase of the absorbance of carbonyl groups of the value 1.0. The values found are included in Table 8.

Table 8

| Stabilizer tested | Xenotest 450 $\Delta A_{CO}$ = 1,0/hr/ |
|---|---|
| 2-hydroxy-4-octyloxybenzophenone | 3 600 |
| Compound according to Example: 9 | 4 700 |
| 13 | 5 900 |
| 18 | 5 300 |
| 22 | 5 100 |

Example 32

The unstabilized high-density polyethylene with the melt flow index $MFI_{190}$ = 6 g/10 min. was blended in the chamber of the Brabender Plastograph at 175 °C within 10 minutes in the nitrogen atmosphere with 0,05 % by weight of 2,6-di-tert.butyl-4-methylphenol, 0,05 % by weight of a stabilizer tested and 0,1 % by weight of calcium stearate. From the such prepared compositions were molded at 200 °C the films of the thickness of 0,1 mm. The films were exposed in the Xenotest 450 to the increase of the absorbance of carbonyl groups of the value 0,1 /hours/. The values found are introduced in Table 9.

Table 9

| Stabilizer tested | % by weight | Xenotest 450 $\Delta A_{CO}$ = 0,1/hr/ |
|---|---|---|
| Individual unstabilized polyethylene | - | 800 |
| Polyethylene without a light stabilizer | - | 1 100 |
| Compound according to Example: 1 | 0,20 | 5 300 |
| 11 | 0,05 | 1 800 |
| 11 | 0,20 | 4 900 |
| 11 | 0,80 | 6 800 |
| 11 | 2,00 | 10 000 |
| 12 | 0,20 | 4 850 |
| 13 | 0,20 | 4 700 |
| 13 | 1,00 | 7 600 |
| 19 | 0,20 | 4 600 |
| 20 | 0,20 | 4 400 |
| 21 | 0,20 | 4 500 |

Example 33

The unstabilized high molecular weight polyethylene with the melt flow index $MFI_{190}$ = 0,14 g/10 min. was blended in the chamber of the Brabender Plastograph at 190 °C within 10 minutes in the nitrogen atmosphere with 0,2 to 0,5 % by weight of a stabilizer tested and 0,1 % by weight of calcium stearate. From the such prepared compositions were molded at 220 °C the films of the thickness of 0,1 mm. The films were exposed in a draft oven at 100 °C and in the Xenotest 450. The time /hours/ , needed for the attainment of the increase of the absorbance of carbonyl groups of the value 0,1 in the draft oven and of the value 0,3 in the Xenotest 450, was investigated. The values obtained are summarized in table 10.

Table 10

| Stabilizer tested | % by weight | Draft oven at 100 °C $\Delta A_{CO}$ = 0,1/hr/ | Xenotest 450 $\Delta A_{CO}$ = 0,3/hr/ |
|---|---|---|---|
| Polyethylene without a stabilizer | - | 150 | 800 |
| Compound according to Example: 2 | 0,2 | 1 000 | 4 700 |
| 7 | 0,2 | 1 500 | 5 000 |
| 13 | 0,2 | 3 000 | 5 200 |
| 13 | 0,5 | >5 000 | >8 000 |
| 19 | 0,2 | 4 700 | 5 600 |
| 20 | 0,3 | 5 200 | 6 100 |

Example 34

The unstabilized linear low-density polyethylene with the melt flow index $MFI_{190}$ = 1,4 g/10 min. was blended with stabilizers tested and from the such compositions were prepared the granulates by using the extruder "Werner Pfleiderer ZSK 30" at 165/175/185/180 °C and 200 rpm. These were processed into the films of the thickness of 0,1 mm by using the equipment "Buzuluk" with the diameter 32 mm at 165 to 185 °C and 100 rpm. The films were exposed in the Xenotest 450 while the time /hours/, needed for the attainment of the increase of the absorbance of carbonyl groups of the value 1,0, was investigated. The values found are listed in Table 11.

Table 11

| Stabilizer tested | % by weight | Xenotest 450,$\Delta A_{CO}$/hr/ |
|---|---|---|
| Polyethylene without a stabilizer | - | 1 500 |
| Compound according to Example: 13 | 0,05 | 2 000 |
| 13 | 0,25 | 5 700 |
| 13 | 1,00 | >8 000 |
| 1 | 0,20 | 5 300 |
| 18 | 0,20 | 4 900 |

Example 35

The unstabilized ethylene-vinylacetate copolymer /5 % of vinylacetate/ with melt flow index $MFI_{190}$ = 2 g/10 min. was blended with stabilizers tested and the compositions obtained were processed into the granulates by using the extruder "Werner-Pfleiderer" at 175 to 195 °C and 100 rpm. From these were formed the films of the thickness of 0,08 mm by using the equipment "Buzuluk" with the diameter 32 mm at 175 to 195 °C and 120 rpm. The photostability of the films was evaluated in the Xenotest 1200 and outdoor weathering. As the criterion of the evaluation was used the decrease of the elongation of break of the films by the influence of the irradiation to 50 % of the origin value / $t_{50}$ /. The values measured are summarized in Table 12.

Table 12

| Stabilizer tested | % by weight | Xenotest 1 200 $t_{50}$/hr/ | Outdoor weathering $t_{50}$/months/ |
|---|---|---|---|
| EVA-copolymer without a stabilizer | - | 760 | 2,5 |
| Compound according to Example: 13 | 0,1 | 1 100 | 6 |
| 13 | 0,2 | 1 300 | 8 |
| 13 | 0,3 | 1 600 | 9,5 |
| Poly-/N-beta-hydroxy-ethyl-2,2,6,6-tetramethyl-4-hydroxypiperidyl/-succinate | 0,2 | 860 | 4 |

Example 36

It has been prepared 5 % by weight of the chloroform solution of polystyrene with the melt flow index $MFI_{200}$ = 16,5 g/10 min. to which were added stabilizers tested in the amount of 0,5 % by weight. The solutions obtained were after mixing poured into the Petri dish. The films of the thickness of 0,05 mm were obtained by the evaporation of chloroform and these were exposed in the Xenotest 450 up to the degradation. The time /hours/ needed for the degradation of samples /t/ is introduced in Table 13.

Table 13

| Stabilizer tested | % by weight | Xenotest 450 t/hr/ |
|---|---|---|
| Polystyrene without a stabilizer | - | 500 |
| Compound according to Example: 3 | 0,5 | 1 200 |
| 17 | 0,5 | 1 400 |
| 18 | 0,5 | 1 500 |
| 21 | 0,5 | 1 550 |
| 22 | 0,5 | 1 480 |

Example 37

ABS copolymer with the melt flow index $MFI_{220}$ = 1 812 g/10 min. was blended with stabilizers tested in the amount of 0,5 % by weight. From the compositions were made the films of the thickness of 0,1 mm and these were exposed by using of Hg-lamp /400 W/ equiped with the Pyrex filter. The induction period $\tau$/hours/ corresponds to A 1 745/A 1 940 of IR spectroscopy. Table 14 comprises the values found.

Table 14

| Stabilizer tested | $\tau$/hr/ |
|---|---|
| ABS copolymer without a stabilizer | 30 |
| Compound according to Example 12 | 80 |
| Compound according to Example 13 | 100 |
| 2-/2-hydroxy-5-methylphenyl/benzotriazole | 60 |

Example 38

To the unstabilized polycarbonate with the melt flow index $MFI_{300}$ = 8,6 g/10 min. were added 0,1 % by weight of 3,5-di-tert. butyl-4-hydroxyphenyloctadecylpropionate, 0,1 % by weight of tris-/2,4-di-tert.butylphenyl/ phosphite and 0,3 % by weight of a light stabilizer. The compositions were processed on the extruder "Werner Pfleiderer" at 248/275/258/225/256 °C and 187 rpm, and from them were molded the films of the thickness of 0,5 mm. The yellowing factor /YF/ was evaluated according to the relation:

$$YF = \frac{\Delta T/420/ - \Delta T/680/}{T_{560}} \cdot 100$$

where $\Delta T$ - is the decrease of the transmission during the irradiation in the Xenotest 450 in the wavelenghts introduced and $T_{560}$ is the value of the transmission /%/ of the nonirradiated sample. The values found are included in Table 15.

Table 15

| Stabilizer tested | YF after the irradiation in the Xenotest 450 | |
|---|---|---|
| | after 5 000 hr | after 9 000 hr |
| 2-/2-hydroxy-5-methylphenyl/benztriazole | 2,5 | 5,2 |
| Compound according to Example 1 | 2,4 | 5,3 |
| Compound according to Example 13 | 2,2 | 5,0 |
| Polycarbonate without a stabilizer | 5,2 | 11,0 |

**Claims**

1. New polyaminotriazines of the general formula (I) :

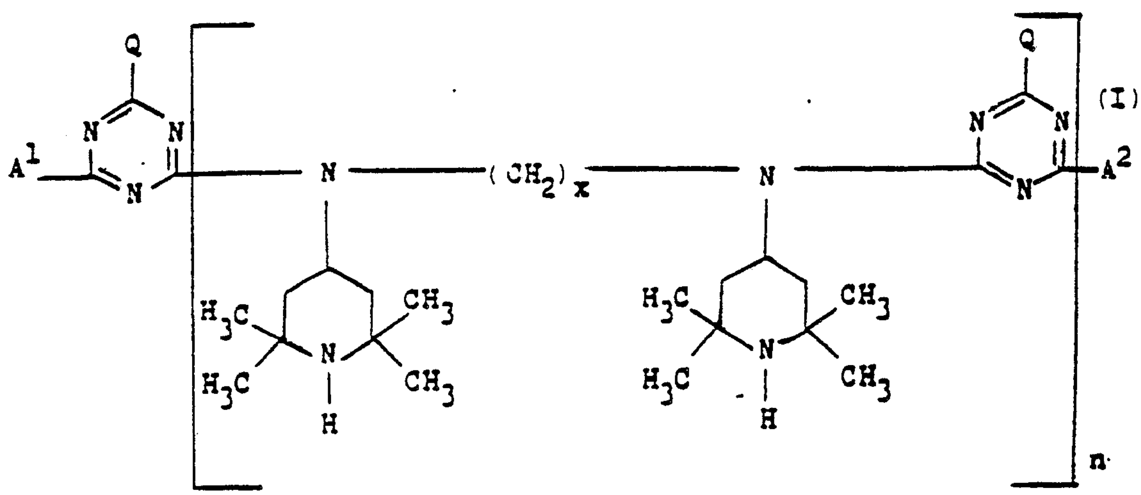

(I)

where Q, $A^1$ and $A^2$ are the same or different and mean the group $-NR^1R^2$, where $R^1$ is alkyl of 1 to 18 carbons, cyclohexyl or 2,2,6,6-tetramethyl-4-piperidyl; $R^2$ is hydrogen, alkyl of 1 to 12 carbons, cyclohexyl, and Q can also mean 1-piperidyl or 1-pyrrolidinyl; x is an integer from 2 to 12 and n is an integer from 2 to 20.

2. A process of the preparation of the polyaminotriazines according to claim 1, wherein N,N′-bis(2,2,6,6-tetramethyl-4-piperidyl)-alkane diamine of the general formula (II):

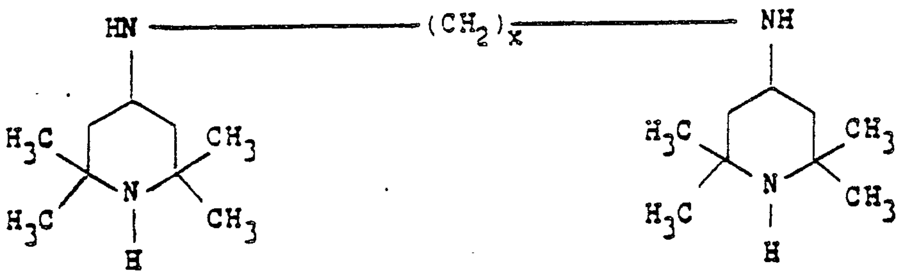

/II/

where x is as defined for formula (I), is left to react with a 6-substituted 2,4-dichloro-1,3,5-triazine of the general formula (III):

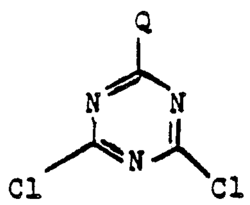

(III)

where Q is as defined in formula (I) in claim 1,
which is used in the excess from 1 to 10 % by mol at 140 to 220 °C in a hydrocarbon solvent in the presence of an aqueous solution of an alkaline hydroxide under elevated pressure, or in the presence of a particulate alkaline hydroxide under atmospheric pressure, for 1 to 12 hours, followed in the formation of an oligomer of the general formula (I) where Q, x and n are as defined in claim 1, and $A^1$ and $A^2$ mean chlorine, which is then treated under the same conditions with an amine of the general formula (IV) :

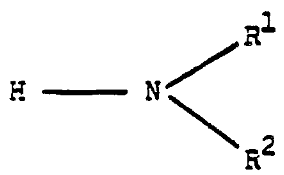

/ IV /

where $R^1$ and $R^2$ are as defined for formula (I) in claim1.

3. The use of a compound according to claim 1 as a light stabiliser in a synthetic organic polymer.